# EUROPEAN PATENT APPLICATION

(11) **EP 1 391 447 A1**
(43) Date of publication of application: **25.02.2004**
(21) Application number: 03254911.5
(22) Date of filing: 07.08.2003
(51) Int. Cl.: C07C 17/16, C07C 22/04, C08F 8/00

(54) **Resin functionalization method**

(30) Priority: 19.08.2002 US 404401 P
(71) Applicant: ROHM AND HAAS COMPANY, Philadelphia, Pennsylvania 19106-2399 (US)
(72) Inventor: Bohling, James Charles, Lansdale, Pennsylvania 19446 (US); Zabrodski, William Joseph, Lansdale, Pennsylvania 19446 (US)
(74) Representative: Kent, Venetia Katherine

(57) **Abstract**

The present invention relates to a method for converting a substituted or unsubstituted solid supported trityl alcohol to a substituted or unsubstituted solid supported trityl chloride.

## Description

### BACKGROUND OF THE INVENTION

Functionalized solid supports have important industrial applications. For example, functionalized solid supports are useful in water treatment for municipalities, private homes and public buildings such as schools and hospitals, and in the semiconductor industry where ultra high purity water is an issue. Further , functionalized solid supports are useful in preparing pharmaceutical dosage formulations and in synthesizing biopharmaceuticals such as therapeutic peptide and proteins.

Of particular importance is the solid support functionalized with a 2'chlorotrityl chloride functionality. Known to those skilled in the art as CTC resins, said resins are useful in therapeutic peptide synthesis and command a very large price. Currently 2'CTC can be purchased from Sigma-Aldrich at a price of $25,000-45,000/Kg in the 1-25g scale. (Sigma-Aldrich Combinatorial Chemistry Catalog, 2001-2002 pg 60.) The advances in peptide therapy are requiring larger, reasonably priced quantities of the CTC resin to synthesize commercial quantities of therapeutic peptides. Thus, there is a need for larger than gram scale quantities of the CTC resin.

The art has attempted to improve on the techniques for preparing CTC solid supports. See, The Advanced Chem Tech Handbook, William D. Bennett et al., 1998 at pg 341 which suggests using 2 eq of pyridine to thionyl chloride (SOCl2). Pyridine is both toxic and foul smelling. Orosz et al. report the use of an excess of trimethylsilylchloride and dimethylsulfoxide followed by treatment with AcCl. See, Orosz et al. Tetrahedron Letters 39 (1998) 3241-3242. The Orosz process is expensive. It also requires extensive washes to remove the dimethylsulfoxide (DMSO) residue. Harre et al. discloses washing the resin with an excess of N, N-dimethylformamide ( DMF) then dichloromethane (DCM)and then treating with SOCl2. Harre also points out the problems associated with this particular reaction. See, Harre et al. Reactive and Functional Polymers 41 (1999) 111-114. Sanghvi et al( US 6239220) discloses, in Example 1 , the conversion of dimethoxytritylalcohol to dimethoxytrityl chloride using acetyl chloride (AcCl). Webber et al.

(US 5563220), in Example 2 ,describes the overnight use of 2 chlorobenzoylchloride to form the keto resin, which is subsequently converted to the alcohol resin. Said alcohol resin is converted to the chloride form by treatment with acetyl chloride. Hodgson et al ( EP0200403A1) describes the use of catalytic amounts of DMF to convert alkoxy alcohols to alkoxy chlorides. No disclosure regarding the converting of non-alkoxy alcohols is made. Ettl et al. (EP0645357A1) describes the use of thionyl chloride or phosgene with dialkyl formamide to convert secondary alkyl alcohols to secondary alkyl chlorides. Ettl discloses a 1 to 1 adduct of DMF and a halogenating agent to form the chlorinating agent. Catalytic amounts of DMF are not disclosed. Nagle et al. in Tetrahedron Letters 41 (2000), 3011-3014 discloses the use of thionyl bromide and a catalytic amount of DMF (0.5-0.6 eq.) for converting β aminoalcohols. This reference teaches that this catalyst works well in non-polar solvents but not in polar solvents such as dichloromethane and that a β amine must be present for this reaction to be improved with DMF.

Applicants have discovered a method for producing solid supported CTC that overcomes the problems in the art and meets the demand for a robust process to produce large scale quantities. Further, the method of the present invention uses materials that are less toxic and foul smelling at catalytic levels.

The term "solid support", as used herein, means an insoluble material that can be functionalized.

The term " functionalization", as used herein, means the addition of a specific moiety to a site on a solid support in order to impart specific properties to said polymeric support.

The term "catalytic amount", as used here in means less than 1 equivalent but more than 0.000001 equivalent.

The term "equivalent" (abbreviated . eq.) as used herein , means the number of moles of functional groups to be transformed.

The term "trityl" is synonymous with the term triphenylmethyl.

### SUMMARY OF THE INVENTION

The present invention relates to a method for converting a substituted or unsubstituted solid supported trityl alcohol to substituted or unsubstituted solid supported trityl chloride comprising the steps:
a. dispersing an organic solvent and a substituted or unsubstituted solid supported trityl alcohol in a reaction vessel;
b. adding 0.00001 to 1.00 equivalents of an amide containing catalyst compound to said reaction vessel;
c. adding a halogenating agent to said reaction vessel;
d. filtering and draining the result of steps a,b, and c to obtain the substituted or unsubstituted solid supported trityl chloride.

### DETAILED DESCRIPTION OF THE INVENTION

The present invention relates to a method for converting a substituted or unsubstituted solid supported trityl alcohol to substituted or unsubstituted solid supported trityl chloride comprising the steps:
a. dispersing an organic solvent and a substituted or unsubstituted solid supported trityl alcohol in a reaction vessel;
b. adding 0.00001 to 1.00 equivalents of an amide containing catalyst compound to said reaction vessel;
c. adding a halogenating agent to said reaction vessel;
d. filtering and draining the result of steps a.,b., and c. to obtain the substituted or unsubstituted solid supported trityl chloride.

The order of addition of the reactants used in steps a., b., and c. is not material to the successful practice of the method of the present invention.

The method of the present invention is run in an organic solvent. Organic solvents useful in the practice of the present invention include, but are not limited to, dichloromethane, toluene, dichloroethane, tetrahydrofuran, benzene, xylene, chloroform and mixtures thereof. More preferred organic solvents are selected from dichloromethane, toluene, dichloroethane , tetrahydrofuran and mixtures thereof. Most preferred organic solvents are selected from dichloromethane and toluene and mixtures thereof.

The solid support is the material that will be functionalized. First said solid support will have an alcohol functionality, and then the alcohol functionality will be converted to the desired chloride form according to the method of the present invention. Solid supports useful in the practice of the present invention include, but are not limited to, controlled pore glass, silica based material, alumina base material, cross linked polystyrene, cross-linked polyacrylates, cross-linked polyamides. More preferred solid supports are selected from the group consisting of cross linked polystyrene and silica based material. The most preferred solid support is cross linked polystyrene.

Solid supported trityl alcohol can be commercially obtained from Aldrich Chemical , or it can be synthesized by methods known to those skilled in the art. See for example Orosz, Tetrahedron Letters, (39 ) 1998 at pg 3241-3241 wherein a cross-linked polystyrene bead can be converted to a polymer supported benzophenone with benzoyl chloride and a Lewis acid catalyst. The resultant benzophenone functionality is then transformed to the trityl alcohol functionality with phenyl magnesiumbromide. The solid supported trityl alcohol is now ready to be converted via the process of the present invention to the solid supported trityl chloride.

The solid supported trityl alcohol ,used to synthesize the solid supported trityl chloride, can be substituted or unsubstituted at any location on the trityl moiety. Said substituents include , but are not limited to, halogens , including but not limited to chloro, bromo and, fluro; substituted or unsubstituted alkoxy groups including but not limited to ethoxy, methoxy, propyloxy, methyleneoxy, ethyleneoxy, ethylene glycol, and propanediol; substituted or unsubstituted alkyl poly ether groups, including ,but not limited to, diethyleglycol, dipropanediol, triethyleneglycol, and tripropyleneglycol; substituted or unsubstituted lower alkyl groups having 1-6 carbon atoms including but not limited to methyl, ethyl, n-propyl, and i-propyl; substituted or unsubstituted aryl groups including, but not limited to, phenyl, benzyl, tolyl, methoxyphenyl, and chlorophenyl, substituted or unsubstituted heteroaryls , and substituted or unsubstituted cycloalkanes. More preferred substituents are methoxy, ethoxy and chloro. The most preferred substituent is chloro.

Amide containing catalyst compounds useful in the practice of the present invention include, but are not limited to N,N-dimethylformamide (DMF), N-methyl-N-ethylformamide, N,N-dimethylacetamide, N-methyl-N-ethylacetamide, and N,N-diethylformamide, other substituted amides and mixtures thereof. More preferred amide containing compounds are N,N-dimethyl formamide and N-methyl-N-ethylformamide and mixtures thereof. The most preferred amide containing compounds is N,N-dimethyl formamide.

The preferred range of amide containing catalyst compound is 0.00001 to 1.00 eq, the more preferred range is 0 .0001 to 0.25 eq , and the most preferred range is 0.1 to 0.001eq

Halogenating agents useful in the practice of the present invention include, but are not limited to phosgene, thionyl chloride, oxalyl chloride, acetyl chloride, phosphorus pentachloride, thionyl bromide and mixtures thereof. More preferred halogenating agents are thionyl chloride and phosphorus pentachloride and mixtures thereof. The most preferred halogenating agent is thionyl chloride.

The following non limiting examples illustrate the practice of the present invention.

### Example 1.

The trityl alcohol resin is added to a round bottom flask containing methylene chloride at 10ml/g resin. The mixture is stirred for 15 minutes. Thionyl chloride (SOCl₂) (2 equivalents based on the level of alcohol incorporated in the resin) is added drop wise followed by DMF (0.04 equivalents based on the level of alcohol incorporated in the resin). The mixture is warmed to 38°C over 15 minutes and held for 4 hours. The mixture is then cooled to ambient temperature and the trityl chloride product is isolated.

### Example 2.

The trityl alcohol resin is added to a round bottom flask containing methylene chloride at 10ml/g resin. DMF (0.04 equivalents based on the level of alcohol incorporated in the resin on a weight/weight percent) is added and the resin is stirred for 15 minutes. Thionyl chloride (5 equivalents) based on the level of alcohol incorporated in the resin) is added drop wise. The mixture is warmed to 38°C over 15 minutes and held for 4 hours. The mixture is then cooled to ambient temperature and the trityl chloride product is isolated.

### Example 3.

The trityl alcohol resin is added to a round bottom flask containing methylene chloride at 10ml/g resin. N-ethyl-N-methylformamide (0.09 equivalents based on the level of alcohol incorporated in the resin) is added and the resin is stirred for 15 minutes. Thionyl chloride (1.1 equivalents) based on the level of alcohol incorporated in the resin) is added drop wise. The mixture is warmed to 38°C over 15 minutes and held for 4 hours. The mixture is then cooled to ambient temperature and the trityl chloride product is isolated.

### Example 4.

The trityl alcohol resin is added to a round bottom flask containing toluene at 10ml/g resin. DMF (0.016 equivalents based on the level of alcohol incorporated in the resin) is added and the resin is stirred for 15 minutes. Oxalyl chloride (2 equivalents) based on the level of alcohol incorporated in the resin) is added drop wise. The mixture is warmed to 38°C over 15 minutes and held for 4 hours. The mixture is then cooled to ambient temperature and the trityl chloride product is isolated.

## Claims

1. A method for converting a substituted or unsubstituted solid supported trityl alcohol to a substituted or unsubstituted solid support trityl chloride comprising the steps:
a. dispersing an organic solvent and a substituted or unsubstituted trityl solid supported alcohol in a reaction vessel;
b. adding 0.00001 to 1.00 equivalents of an amide containing catalyst compound to said reaction vessel;
c. adding a halogenating agent to said reaction vessel;
d. filtering and draining the result of steps a,b, and c to obtain the substituted or unsubstituted trityl chloride solid support.

2. A method according to Claim 1, wherein said organic solvent is selected from the group consisting of dichloromethane, toluene, dichloroethane, and tetrahydrofuran, and mixtures thereof.

3. A method according to Claim 2, wherein said amide containing catalyst is selected from the group consisting of N,N-dimethylformamide and N-methyl-N-ethylformamide and mixtures thereof.

4. A method according to Claim 3, wherein said halogenating agent is selected from the group consisting of thionyl chloride and phosphorus pentachloride and mixtures thereof.

5. A method according to Claim 4, wherein said amide containing catalyst is N,N-dimethylformamide , further provided that .0001 to .25 equivalents of said N,N-dimethylformamide are added to said reaction vessel.
